# EUROPEAN PATENT SPECIFICATION

(11) **EP 1 509 140 B1**
(45) Date of publication and mention of the grant of the patent: **13.05.2009**
(21) Application number: 03756317.8
(22) Date of filing: 30.05.2003
(51) Int. Cl.: A61B 10/00, A61B 17/34

(54) **APPARATUS TO ACCESS THE BONE MARROW**
GERÄT FÜR DEN ZUGANG ZU KNOCHENMARK
DISPOSITIF PERMETTANT D'ACCEDER A LA MOELLE OSSEUSE

(30) Priority: 31.05.2002 US 384756 P
(43) Date of publication of application: 02.03.2005
(60) Divisional application: 09155111.9
(73) Proprietor: Vidacare Corporation, San Antonio, TX 78216 (US)
(72) Inventor: MILLER, Larry, J., Spring Branch, TX 78070 (US)
(74) Representative: Ljungberg, Robert
(86) International application number: PCT/US2003/017167
(87) International publication number: WO 2003/101306

(56) References cited:
- EP-A- 0 807 412
- WO-A-93/07819
- WO-A-96/31164
- WO-A-98/06337
- FR-A- 853 349
- GB-A- 2 130 890

## Description

### TECHNICAL FIELD

The present invention is related to an apparatus for withdrawing specimens from the bone or bone marrow. The apparatus can be used to extract stem cells or bone marrow for transplantation or diagnostic purposes.

### BACKGROUND OF THE INVENTION

There are many clinical conditions where it is important to be able to access and retrieve bone marrow. In some cases it may be necessary to treat diseases with a bone marrow or stem cell transplant to restore functioning blood cells in the body after high-dose chemotherapy. Such conditions may include acute leukemias, brain tumors, breast cancer, Hodgkin's disease, multiple myeloma, neuroblastoma, non-Hodgkin's lymphomas, ovarian cancer, sarcoma and testicular cancer. In other cases it is necessary to access the bone marrow to obtain a sample of the marrow for diagnostic testing. These conditions may include cancer of any type and hematologic disease of any origin.

Current techniques for gaining access to bone marrow can be difficult, traumatic and occasionally dangerous, depending on the site selected for harvest, the operator's expertise and the patient's anatomy. In general, the devices available for gaining access to the medullary cavity of the bone, where the bone marrow is located, include a trocar-fitted needle, with handles to facilitate application of pressure and rotation. These types of devices require substantial force to break through the outer cortex of the bone by a fracturing technique. The exertion of high pressure upon the needle causes pain for the patient and often damages the tip of the needle. This is particularly a problem when harvesting from the sternum of a patient because the excess force can cause penetration through the sternum and can damage underlying structures such as the heart and great vessels.

Another disadvantage of current techniques to access the bone marrow is that frequently more than one penetration site into the bone is required to retrieve enough bone marrow to either perform diagnostic tests or for transplantation purposes. To retrieve an adequate sample of bone marrow for either a bone marrow or stem cell transplant, a physician may need to put the needle into several different parts of the pelvis which may require up to six needle puncture sites or more. This multiple-pass requirement can be extremely painful for a patient and may deter people from donating bone marrow. This technique of using multiple passes can also cause fatigue in smaller operators who may lack strength to complete multiple-pass procedures.

Retrieving bone samples for diagnostic purposes is likewise difficult. Occasionally the core sample of bone is not retrieved because it is not extracted successfully with a standard biopsy needle. Thus, multiple attempts may be necessary to obtain a satisfactory bone or bone marrow biopsy.

Current techniques require that biopsy needles be forced by manual pressure into bone. These techniques may have undesirable side effects such as damaging a needle tip or having the needle slide off the proposed bone target and into organs or soft tissues.

WO980633 discloses a hard tissue drug delivery device and method that perforates hard tissue and provides a drug delivery passage into underlying tissue.

FR 853349 discloses a device for obtaining a sample of tissue from human organs or animals.

### SUMMARY OF THE INVENTION

There is a need for an apparatus and method to access the bone marrow that is minimally traumatic to the patient and that allows a sufficient amount of bone marrow to be removed the first time the bone is penetrated. In accordance with teachings of the present invention, an apparatus and method for the removal of portions of bone marrow from a bone are provided.

The invention provides an apparatus according to claim 1, and a hollow drive shaft assembly according to claim 16.

In one embodiment the apparatus includes a housing and a hollow drive shaft operable to engage a gear assembly. The hollow drive shaft includes a first end operable to penetrate the bone and a second end operable to allow retrieval of portions of bone and bone marrow. The hollow drive shaft also includes an inner channel operable to convey portions of bone and bone marrow. The apparatus also includes a removable trocar with a first end operable to penetrate the bone and a second end. The removable trocar includes an inner channel operable to convey portions of bone and bone marrow. The gear assembly is able to engage and rotate the hollow drive shaft. A motor operable to engage the gear assembly and drive the hollow drive shaft into the bone marrow by rotation of the hollow drive shaft and a power supply and associated circuitry operable to power the motor are also included.

In a further embodiment of the invention a hollow drive shaft assembly operable to remove bone or tissue from a bone marrow is provided. An outer hollow drive shaft and a removable inner trocar operable to penetrate the bone marrow are included. The outer hollow drive shaft is removably coupled to a gear assembly and the outer hollow drive shaft includes an inner channel operable to convey portions of bone and bone marrow.

In a particular embodiment of the invention, the site of harvest within the bone marrow may be changed by a ratcheted gear attached to the second end of the trocar. The gear may be used to change the depth of sampling from the bone marrow.

### BRIEF DESCRIPTION OF THE DRAWINGS

A more complete and thorough understanding of the present embodiments and advantages thereof may be acquired by referring to the following description taken in conjunction with the accompanying drawings, in which like reference numbers indicate like features, and wherein:
FIGURE 1A illustrates an embodiment of an apparatus for removing a bone marrow sample shown in longitudinal cross section.
FIGURE 1B illustrates an embodiment of a hollow drive shaft of the current invention.
FIGURE 1C illustrates an embodiment of a trocar of the current invention.
FIGURE 1D illustrates an embodiment of an apparatus of the current invention.
FIGURE 1E illustrates an embodiment of an apparatus for removing a bone marrow sample shown in longitudinal cross section.
FIGURE 2A illustrates an example of a hollow drive shaft or penetrator of the current invention.
FIGURE 2B illustrates an example of a hollow drive shaft or penetrator of the current invention.
FIGURE 2C illustrates an example of an inner trocar of the current invention.
FIGURE 2D illustrates an example of a hollow drive shaft or penetrator of the current invention.
FIGURE 3A illustrates an example of an attachment of the current invention.
FIGURE 3B is an illustration of an attachment of the current invention.
FIGURE 4A is an illustration of a hollow drive shaft and trocar of the current invention.
FIGURE 4B is an illustration of a hollow drive shaft and trocar of the current invention.
FIGURE 4C is an illustration of a hollow drive shaft and trocar of the current invention in cross section.
FIGURE 4D is an illustration of a hollow drive shaft and trocar of the current invention in cross section.
FIGURE 5A-C illustrates example gear assemblies of the current invention.

### DETAILED DESCRIPTION OF THE INVENTION

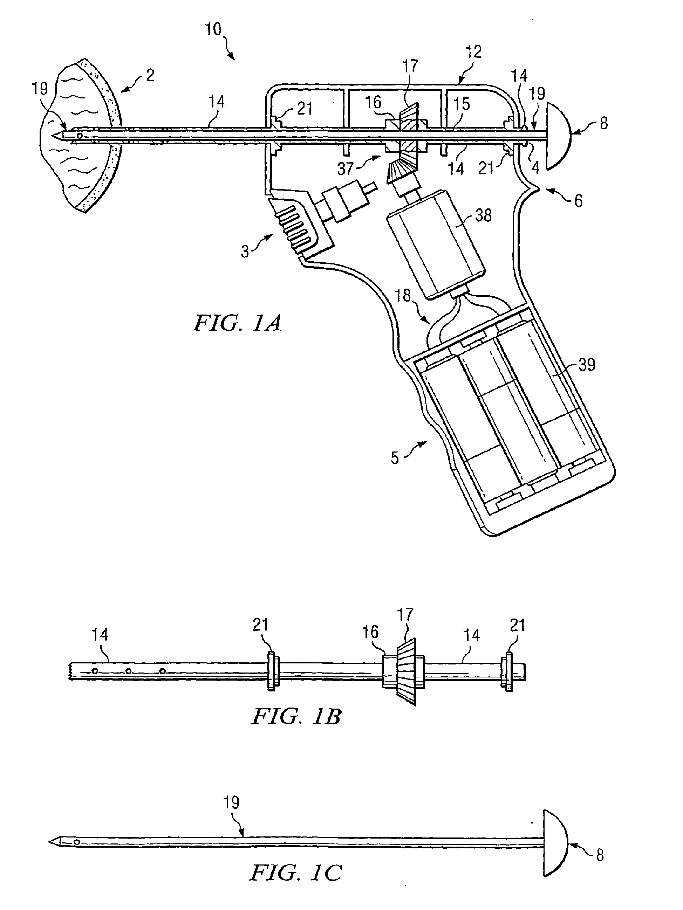

Preferred embodiments of the invention and its advantages are best understood by reference to FIGURES 1A-5C herein like numbers refer to same and like parts.

FIGURE 1A, illustrates an example of an apparatus for removing bone marrow from a bone. Apparatus 10 may be used to obtain a sample of bone marrow from any suitable bone in the body such as the iliac crest or the sternum. In one embodiment, apparatus 10 includes housing 12, a hollow drive shaft 14, removable trocar 19, gear assembly 37, motor 38 and power supply 39. Housing 12 may include on/off switch 3 handle 5 and guard 6. Handle 5 may be angled downward to allow ease of operation and also to allow end of hollow drive shaft 14 to turn without obstruction from a hand. Housing 12 encompasses power source 39, motor 38 and associated circuitry 18, hollow drive shaft 14 and gear assembly 37. Hollow drive shaft 14 includes inner channel 15 and collar 16. Gear 17 of gear assembly 37 engages collar 16 of hollow drill shaft 14 and thereby rotates hollow drive shaft 14.

Removable trocar 19 may be inserted into inner channel 15 of hollow drive shaft 14. Trocar 19 is hollow and has an inner channel operable to convey bone and bone marrow samples. Trocar 19 may have a handle 8 that can be used to tighten trocar 19 in place or to remove it from inner channel 15 of hollow drive shaft 14. Hollow drive shaft 14 may include luer lock connector 4 at the point where it exits housing 12. A luer lock connector may allow hollow drive shaft to connect to a suction apparatus such as a tubing or syringe or to any other suitable apparatus that would assist in obtaining a bone or bone marrow biopsy specimen. An access port, such as a suction port may also releasably attach to an end of hollow drive shaft where it exits housing 12, for example to a luer lock connector. Such an attachment may be a plug, a port, a suction apparatus, swivel port or other adapter.

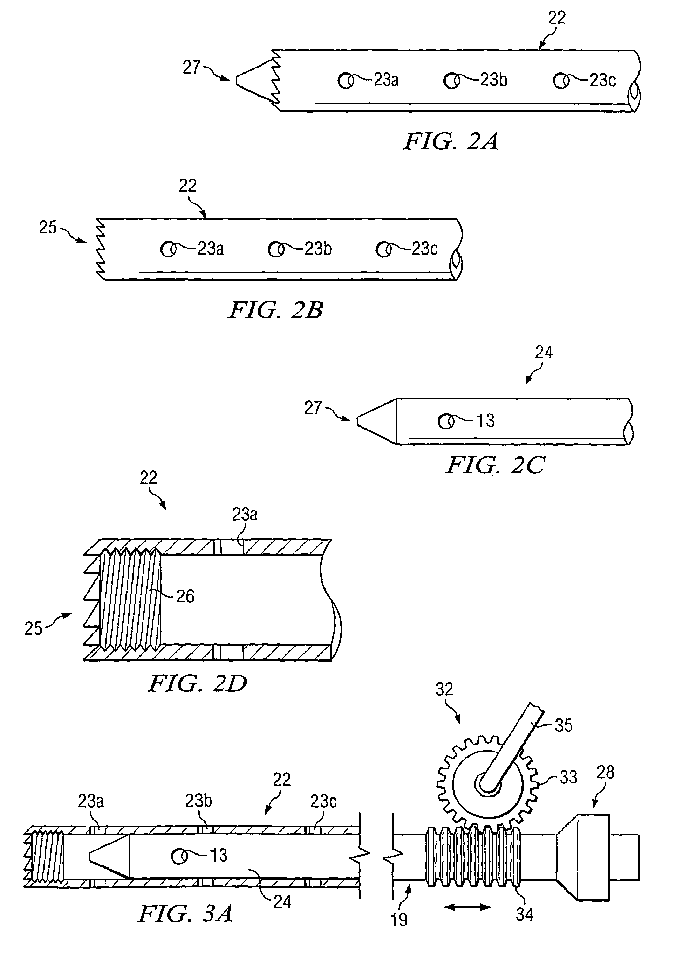

FIGURE 1B shows hollow drive shaft 14 removed from apparatus 10. In one embodiment, hollow drive shaft 14 may include one or more thrust bearings 21. Thrust bearings may absorb pressure from the thrust of a drive shaft into bone during drilling. Also included in hollow drive shaft are side ports shown in further detail in FIGURE 2. FIGURE 1C illustrates removable trocar 19 which includes handle 8. Handle 8 may be formed into a shape that is easily grasped and turned during the process of obtaining a biopsy. Removable trocar 19 may include one or more side ports operable to access a bone marrow specimen shown in greater detail in FIGURE 2.

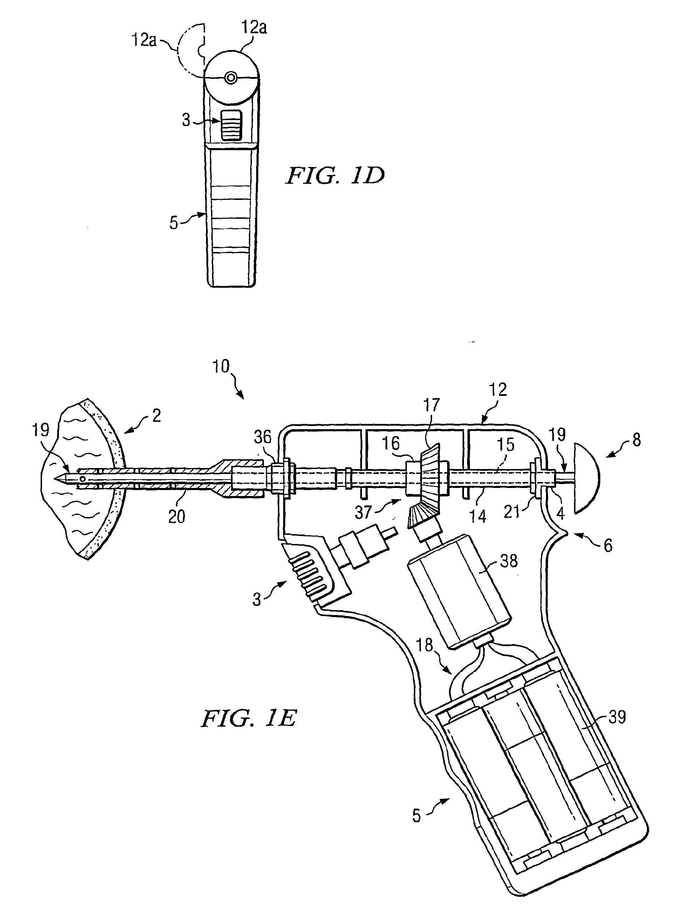

In one embodiment, shown in FIGURE 1D, housing 12 may include a releasable hatch or door 12a that may be opened to allow hollow drive shaft and attached gear 17 to be removed after use of apparatus 10. This may be desirable where apparatus 10 is reusable and hollow drill shaft 14 and removable trocar 19 are disposable. A releasable hatch or door may also be desirable to clean the inside of apparatus 10.

In another embodiment of the current invention, shown in FIGURE 1E, detachable penetrator 20 may be attached to hollow drive shaft 14 by means of connector 36 and drilled into bone 2. In this embodiment, removable trocar 19 may be inserted into inner channel 15 of hollow drive shaft 14 and into the hollow channel of penetrator 20. Penetrator 20 may include side ports to permit access to bone and bone marrow samples during a biopsy or bone marrow harvest procedure. One advantage of an embodiment that includes a detachable penetrator is it allows penetrators of various sizes and configurations to be attached to hollow drive shaft 14. In this embodiment, connector 36 includes an inner channel operable to allow retrieval of bone and bone marrow specimens.

FIGURES 2A-D show an end 22 of either a hollow drive shaft or a penetrator that is suitable to penetrate a bone. FIGURE 2A, illustrates hollow drive shaft or penetrator end 22 which may include multiple sampling ports 23 through which bone marrow or other biopsy material may be aspirated. Sampling ports 23a, 23b and 23c are each operable to retrieve a portion of bone marrow. When removable trocar end 24 is in position within inner channel of hollow drive shaft end 22 sampling port 13 of removable trocar end 24 may align with sampling port 23a, 23b, or 23c. An operator may determine the level in the bone marrow where a specimen is taken or which sampling port (23a, 23b or 23c) to align with trocar sampling port 13. Hollow drive shaft or penetrator end 22 may include serrated tip 25 as shown in FIGURE 2B or any other suitable configuration for sampling bone or bone marrow.

FIGURE 2C shows removable trocar end 24 having a sampling port 23 near tip 27 through which a sample of bone or bone marrow can be retrieved as well as through a sampling port 23a, 23b or 23c. When sampling port 23a, 23b or 23c of hollow drive shaft or penetrator 22 is aligned with sampling port 13 of removable trocar 24, a portion of bone marrow may be suctioned out of the bone. Removable trocar 24 may be removed allowing bone marrow to be suctioned through one or more sampling ports 23 of hollow drive shaft or penetrator 22 at different sites in a bone marrow cavity sequentially. FIGURE 2D shows another example of tip 25 of hollow drive shaft or penetrator end 22 having internal threads 26 that are able to engage a specimen of bone and core it out as hollow drive shaft or penetrator 22 is drilled into bone. Threads 26 may engage and adhere to a portion of bone or to a semisolid substance such as bone marrow and maintain contact with the specimen so it may be successfully retrieved.

FIGURE 3A shows operating mechanism 32 that may be included in some embodiments of the invention and may be attached to hollow drive shaft 14 and manipulated to change the depth in a bone marrow where sampling occurs. Operating mechanism 32 includes handle 35 and gear 33. Gear 33 engages gear 34 attached to trocar 19. Suction port 28, shown attached to trocar 19, may be used to retrieve portions of bone marrow from inner channel 15 of hollow drive shaft 14.

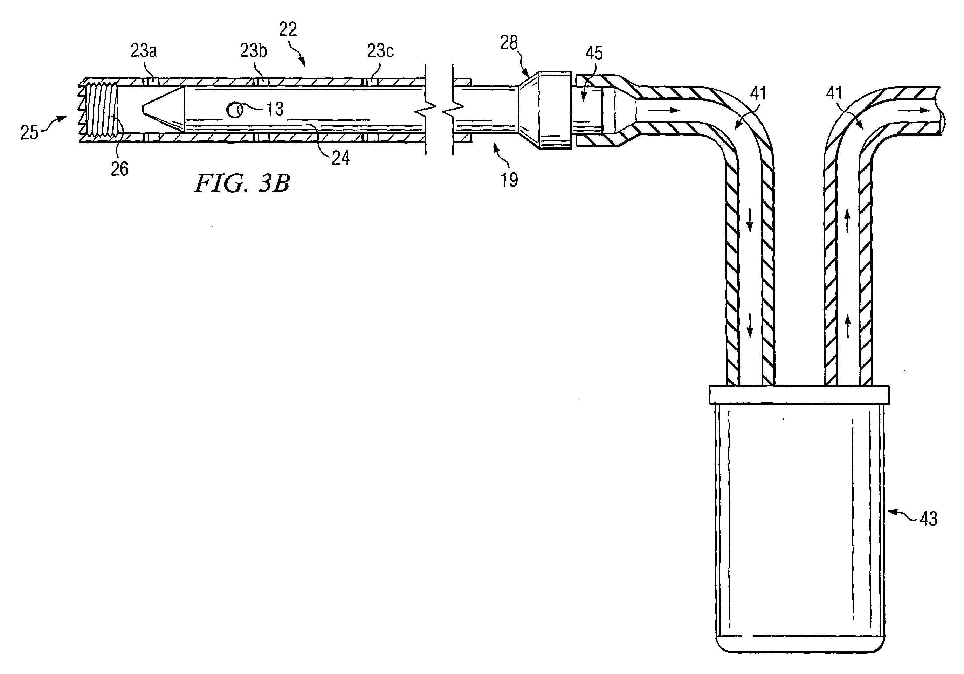

FIGURE 3B shows an example suction port 28 that may be connected to trocar-19 where it exits housing 12. In one embodiment of the invention a suction port or suction swivel apparatus may also be connected to the hollow drive shaft 14. This feature allows a suction apparatus of the type well known to one skilled in the art to be used to obtain bone marrow samples. Suction port 28 may also be configured to attach directly to a penetrator for example in an embodiment of the invention where the penetrator is detached from the housing before it is accessed for bone marrow retrieval. Also shown in FIGURE 3B is a suction swivel apparatus 45 that allows a suction tube 41 to attach to suction port 28. Suction swivel apparatus 45 allows suction port 28 to remain attached to suction tube 41 while an attached drilling apparatus is drilling into bone marrow without kinking or twisting suction tube 41. Also shown in FIGURE 3B is receptacle 43 that is interposed between suction tube 41 and a source of suction such that a bone marrow specimen may be successfully retrieved into receptacle 43 and not lost into a suction machine.

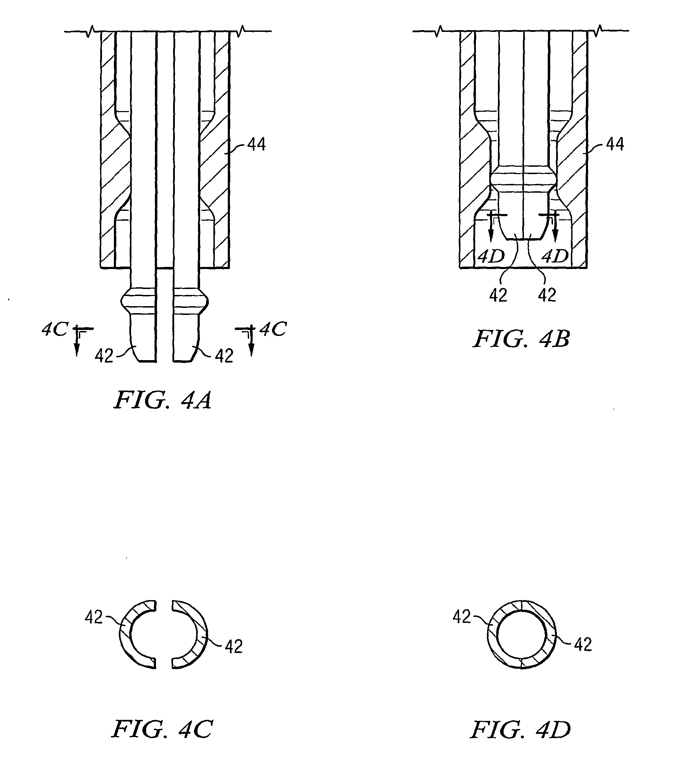

FIGURE 4A-D shows an example hollow drive shaft end or penetrator end 44 and inner trocar 42 having a split-needle configuration. An inner trocar 42 is inserted into a hollow drive shaft or penetrator end 44. Inner trocar 42 shown in the open position in FIGURE 4A is advanced past the end of hollow drive shaft end or penetrator end 44 where it may be opened and inserted into bone, bone marrow or other tissue. Inner trocar 42 may be closed, shown in FIGURE 4B, so that a specimen is retained in its grasp as it is withdrawn from the body. FIGURE 4C shows a cross section of the split-needle inner trocar 42 where the tip of the inner trocar 42 is in the open position. FIGURE 4D shows a cross section of the trocar 42 is shown in the closed position.

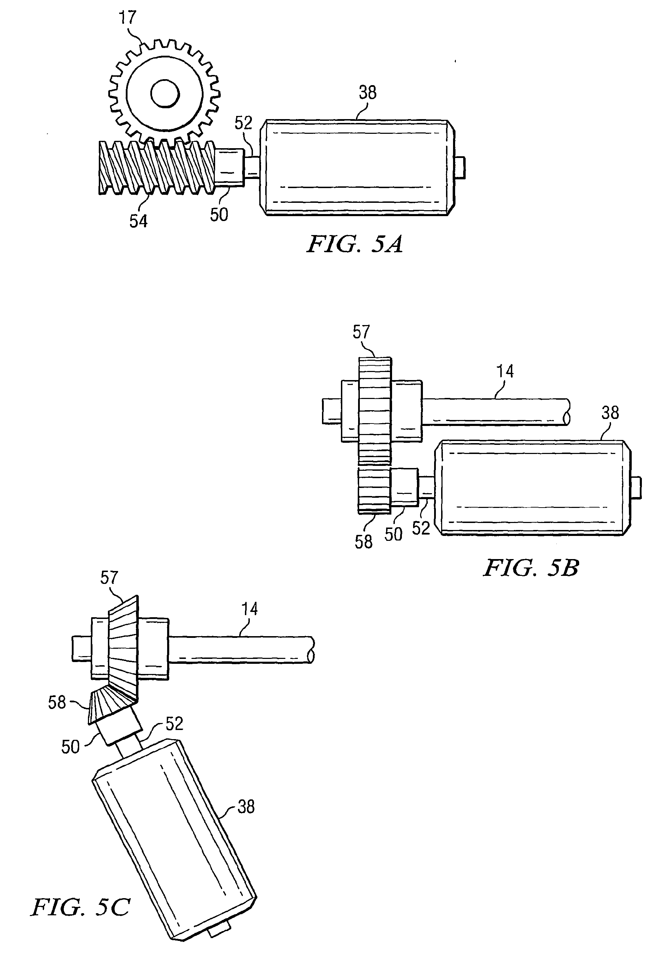

The apparatus may or may not include a reduction gear assembly. A reduction gear assembly may include a worm gear assembly shown in more detail in FIGURE 5A and may include first connector 50 that connects shaft 52 of motor 38 to worm gear 54. A reduction gear assembly may be used to decrease the RPMs between the motor and penetrator assembly to provide an optimum RPM at the point of insertion of penetrator assembly into bone. FIGURE 5B illustrates a further embodiment of a reduction gear assembly wherein a first spur gear 57 engages a second spur gear 58. FIGURE 5C illustrates an alternate embodiment of a reduction gear assembly wherein spur gear 56 is offset forty-five degrees relative to hollow drive shaft 14 which may be preferable in some embodiments of the present invention. In this embodiment spur gear 58 may be offset at any angle and is not limited to forty-five degrees. Other gears may be used in a reduction gear assembly, for example a planetary gear (not expressly shown) may be used alone or in combination with a worm gear or a spur gear.

Although the present invention and its advantages have been described in detail, it should be understood that various changes, substitutions and alternations can be made herein without departing from the scope of the invention as defined by the following claims.

## Claims

1. An apparatus (10) for removing portions of bone and bone marrow from a bone (2) for diagnostic or therapeutic purposes comprising:
a housing (12);
a hollow drive shaft (14) operable to engage a gear assembly (37);
the hollow drive shaft (14) comprising a first end (22) operable to penetrate the bone;
the hollow drive shaft further comprising an inner channel (15) operable to convey portions of bone and bone marrow;
a removable trocar (19) being insertable into the inner channel of the hollow drive shaft (14), said trocar comprising a first end (24) operable to penetrate the bone and a second end;
the gear assembly (37) operable to engage the hollow drive shaft;
a motor (38), coupled to the hollow drive shaft, operable to drive the trocar into the bone marrow by rotation of the hollow drive shaft; and
a power supply (39) and associated circuitry operable to power the motor, **characterized in that** the hollow drive shaft comprises:
a second end operable to allow retrieval of portions of bone and bone marrow, wherein the removable trocar further comprises an inner channel operable to convey portions of bone and bone marrow.

2. The apparatus of Claim 1 wherein the hollow drive shaft (14) and removable trocar (19) are disposable and operable to be removed from the apparatus through a releasably coupled access panel in the housing.

3. The apparatus of Claim 1 wherein the hollow drive shaft (14) is operable to attach to an access port.

4. The apparatus of Claim 1 wherein the hollow drive shaft is operable to attach to a suction port (28).

5. The apparatus of Claim 1 wherein the second end of the trocar (19) is coupled to a ratcheted gear (33) the gear operable to align the sampling ports of the trocar with the sampling ports of the hollow drive shaft.

6. The apparatus of Claim 1 wherein the removable trocar (19) comprises a tip (27) having a longitudinal groove operable to release bone chips from an insertion site into the bone marrow and the outer penetrator includes a cutting edge.

7. The apparatus of Claim 1 wherein the housing (12) comprises a portion of a base mounted on a surface.

8. The apparatus of Claim 1 wherein the hollow drive shaft (14) comprises a tip (25) having internal threads (26) operable to engage a bone or bone marrow biopsy specimen as it is removed from the bone marrow.

9. The apparatus of Claim 1 wherein the removable trocar (19) comprises a tip (27) having two separate longitudinal pieces that can move together or apart.

10. The apparatus of Claim 1 further comprising a reduction gear assembly operable to rotate the hollow drive shaft (14).

11. The apparatus of Claim 1 or Claim 16 wherein the hollow drive shaft (14) comprises at least one thrust bearing (21).

12. The apparatus of Claim 1 wherein the hollow drive shaft (14) comprises at least one side port (23a) operable to access a portion of bone or bone marrow.

13. The apparatus of Claim 1 wherein the removable trocar (19) comprises at least one side port (13) operable to align with a side port (23a) of the hollow drive shaft (14) to access a portion of bone or bone marrow.

14. The apparatus of Claim 1 or Claim 16 wherein the hollow drive shaft (14) comprises a luer lock connector (4).

15. The apparatus of Claim 1 wherein the inner trocar (19) comprises two longitudinal bodies, the longitudinal bodies having tips with jaws operable to grasp a portion of bone or bone marrow and wherein the longitudinal bodies are operable to separate or come together depending on their position relative to the hollow drive shaft (14).

16. A hollow drive shaft assembly operable to remove bone or bone marrow from a bone (2) for diagnostic or therapeutic purposes comprising:
an outer hollow drive shaft (14), and a removable inner trocar (19) operable to penetrate the bone marrow;
the outer hollow drive shaft removably coupled to a gear assembly (37); and
the outer hollow drive shaft further comprising an inner channel (15) operable to convey portions of bone and bone marrow; **characterized by** the removable trocar comprising an inner channel operable to convey portions of bone and bone marrow.

17. The hollow drive shaft assembly of Claim 16 wherein the hollow drive shaft (14) comprises a tip (25) with serrated teeth, the hollow drive shaft further comprising internal threads (26) operable to engage a portion of bone or bone marrow.

18. The hollow drive shaft assembly of Claim 16 wherein the hollow drive shaft (14) comprises a body with at least one side port (23a) operable to obtain portions of bone marrow.

19. The hollow drive shaft assembly of Claim 16 wherein the inner trocar (19) comprises a tip (27) with a cutting edge and further comprises a body with at least one side port (13) operable to obtain bone marrow tissue from the bone marrow.

20. The hollow drive shaft assembly of Claim 16 wherein the inner trocar (19) comprises:
two longitudinal bodies, the longitudinal bodies further comprising tips with curved jaws; and
the longitudinal bodies operable to separate or come together depending on their position relative to the outer penetrator, and further operable to grasp a portion of bone or bone marrow between the curved jaws.

## Patentansprüche

1. Vorrichtung (10) zum Entfernen von Teilen eines Knochens und Knochenmarks von einem Knochen (2) zu diagnostischen oder therapeutischen Zwecken, aufweisend:
ein Gehäuse (12);
eine hohle Antriebswelle (14), ausgebildet zum Angreifen einer Zahnradanordnung (37);
wobei die hohle Antriebswelle (14) ein erstes Ende (22), ausgebildet zum Eindringen in den Knochen, aufweist;
wobei die hohle Antriebswelle weiterhin einen inneren Kanal (15), ausgebildet zum Transportieren von Teilen von Knochen und Knochenmark, aufweist;
einen entfernbaren Trokar (19), welcher in den inneren Kanal der hohlen Antriebswelle (14) einführbar ist,
wobei der Trokar ein erstes Ende (24), ausgebildet zum Eindringen in den Knochen, und ein zweites Ende aufweist;
wobei die Zahnradanordnung (37) zum Angreifen der hohlen Antriebswelle ausgebildet ist;
einen Motor (38), gekoppelt an die hohle Antriebswelle, ausgebildet zum Einfahren des Trokars in das Knochenmark durch Drehung der hohlen Antriebswelle; und
eine Energieversorgung (39) und dazugehörige Schaltungsanordnung, ausgebildet zum Versorgen des Motors, **dadurch gekennzeichnet, dass** die hohle Antriebswelle ein zweites Ende, ausgebildet zur Gewinnung von Teilen von Knochen und Knochenmark, aufweist, wobei der entfernbare Trokar weiterhin einen inneren Kanal, ausgebildet zum Transportieren von Teilen von Knochen und Knochenmark, aufweist.

2. Vorrichtung nach Anspruch 1, wobei die hohle Antriebswelle (14) und der entfernbare Trokar (19) Einwegartikel sind und ausgebildet sind, durch ein lösbar gekoppeltes Zugangselement in dem Gehäuse von der Vorrichtung entfernt zu werden.

3. Vorrichtung nach Anspruch 1, wobei die hohle Antriebswelle (14) zum Befestigen an einem Zugangsanschluss ausgebildet ist.

4. Vorrichtung nach Anspruch 1, wobei die hohle Antriebswelle zum Befestigen an einem Sauganschluss (28) ausgebildet ist.

5. Vorrichtung nach Anspruch 1, wobei das zweite Ende des Trokars (19) an ein Klinkenzahnrad (33) gekoppelt ist, wobei das Zahnrad zum Ausrichten der Entnahmeöffnungen des Trokars auf die Entnahmeöffnungen der hohlen Antriebswelle ausgebildet ist.

6. Vorrichtung nach Anspruch 1, wobei der entfernbare Trokar (19) eine Spitze (27) mit einer Längskerbe aufweist, ausgebildet zum Abgeben von Knochensplittern von einer Ansatzstelle in das Knochenmark, und wobei die äußere Einstechvorrichtung eine Schneidekante aufweist.

7. Vorrichtung nach Anspruch 1, wobei das Gehäuse (12) einen Teil einer Basis aufweist, befestigt auf einer Oberfläche.

8. Vorrichtung nach Anspruch 1, wobei die hohle Antriebswelle (14) eine Spitze (25) mit inneren Gewindegängen (26), ausgebildet zum Angreifen einer Knochen- oder Knochenmarkbiopsieprobe bei deren Entnahme aus dem Knochenmark, aufweist.

9. Vorrichtung nach Anspruch 1, wobei der entfernbare Trokar (19) eine Spitze (27) mit zwei getrennten Längsteilen, die sich zusammen- oder wegbewegen können, aufweist.

10. Vorrichtung nach Anspruch 1, weiterhin aufweisend eine Untersetzungs-Zahnradanordnung, ausgebildet zum Drehen der hohlen Antriebswelle (14).

11. Vorrichtung nach Anspruch 1 oder Anspruch 16, wobei die hohle Antriebswelle (14) zumindest ein Stützlager (21) aufweist.

12. Vorrichtung nach Anspruch 1, wobei die hohle Antriebswelle (14) zumindest eine Seitenöffnung (23a), ausgebildet für den Zugang zu einem Teil eines Knochens oder Knochenmarks, aufweist.

13. Vorrichtung nach Anspruch 1, wobei der entfernbare Trokar (19) zumindest eine Seitenöffnung (13), ausgebildet zum Ausrichten auf eine Seitenöffnung (23a) der hohlen Antriebswelle (14), für den Zugang zu einem Teil eines Knochens oder Knochenmarks aufweist.

14. Vorrichtung nach Anspruch 1 oder Anspruch 16, wobei die hohle Antriebswelle (14) ein Luer-Lock Verbindungsteil aufweist.

15. Vorrichtung nach Anspruch 1, wobei der innere Trokar (19) zwei Längskörper aufweist, wobei die Längskörper Spitzen mit Backen, ausgebildet zum Greifen eines Teils eines Knochens oder Knochenmarks, besitzen, und wobei die Längskörper ausgebildet sind, sich voneinander zu trennen oder zusammenzukommen, abhängig von ihrer Position relativ zu der hohlen Antriebswelle (14).

16. Hohle Antriebswellenanordnung, ausgebildet zum Entfernen von Knochen oder Knochenmark von einem Knochen (2) zu diagnostischen oder therapeutischen Zwecken, aufweisend:
eine äußere hohle Antriebswelle (14) und einen entfernbaren inneren Trokar (19), ausgebildet zum Eindringen in das Knochenmark;
wobei die äußere hohle Antriebswelle lösbar an eine Zahnradanordnung (37) gekoppelt ist; und
wobei die äußere hohle Antriebswelle weiterhin einen inneren Kanal (15), ausgebildet zum Transportieren von Teilen von Knochen und Knochenmark, aufweist; **dadurch gekennzeichnet, dass** der entfernbare Trokar einen inneren Kanal, ausgebildet zum Transportieren von Teilen von Knochen und Knochenmark, aufweist.

17. Hohle Antriebswellenanordnung nach Anspruch 16, wobei die hohle Antriebswelle (14) eine Spitze (25) mit gezackten Zähnen aufweist, und wobei die hohle Antriebswelle weiterhin innere Gewindegänge (26), ausgebildet zum Angreifen eines Teils eines Knochens oder Knochenmarks, aufweist.

18. Hohle Antriebswellenanordnung nach Anspruch 16, wobei die hohle Antriebswelle (14) einen Körper mit zumindest einer Seitenöffnung (23a), ausgebildet zum Erhalten von Teilen von Knochenmark, aufweist.

19. Hohle Antriebswellenanordnung nach Anspruch 16, wobei der innere Trokar (19) eine Spitze (27) mit einer Schneidekante aufweist und weiterhin einen Körper mit zumindest einer Seitenöffnung (13), ausgebildet zum Erhalten von Knochenmarksgewebe von dem Knochenmark, aufweist.

20. Hohle Antriebswellenanordnung nach Anspruch 16, wobei der innere Trokar (19) aufweist:
zwei Längskörper, wobei die Längskörper weiterhin Spitzen mit gekrümmten Backen aufweisen; und
wobei die Längskörper ausgebildet sind, sich voneinander zu trennen oder zusammenzukommen, abhängig von ihrer Position relativ zu der äußeren Einstechvorrichtung, und zudem zum Fassen eines Teils eines Knochens oder Knochenmarks zwischen den gekrümmten Backen ausgebildet sind.

## Revendications

1. Appareil (10) destiné à retirer des portions d'os et de moelle osseuse d'un os (2) à des fins de diagnostic ou thérapeutiques comprenant :
un boîtier (12) ;
un arbre d'entraînement creux (14) pouvant fonctionner pour s'engager sur un ensemble d'engrenages (37) ;
l'arbre d'entraînement creux (14) comprenant une première extrémité (22) pouvant fonctionner pour pénétrer dans l'os ;
l'arbre d'entraînement creux comprenant en outre un canal intérieur (15) pouvant fonctionner pour transporter des portions d'os et de moelle osseuse ;
un trocart amovible (19) pouvant être inséré dans le canal intérieur de l'arbre d'entraînement creux (14), ledit trocart comprenant une première extrémité (24) pouvant fonctionner pour pénétrer dans l' os, et une seconde extrémité ;
l'ensemble d'engrenages (37) pouvant fonctionner pour s'engager dans l'arbre d'entraînement creux ;
un moteur (38) couplé à l'arbre d'entraînement creux pouvant fonctionner pour entraîner le trocart dans la moelle osseuse par rotation de l'arbre d'entraînement creux ; et
une alimentation électrique (39) et des circuits associés pouvant fonctionner pour alimenter en électricité le moteur, **caractérisé en ce que** l'arbre d'entraînement creux comprend :
une seconde extrémité pouvant fonctionner pour permettre l'enlèvement de portions d'os et de moelle osseuse, dans laquelle le trocart amovible comprend en outre un canal intérieur pouvant fonctionner pour transporter des portions d'os et de moelle osseuse.

2. Appareil selon la revendication 1, dans lequel l'arbre d'entraînement creux (14) et le trocart amovible (19) sont jetables et peuvent fonctionner pour être retirés de l'appareil à travers un panneau d'accès couplé de manière détachable dans le boîtier.

3. Appareil selon la revendication 1, dans lequel l'arbre d'entraînement creux (14) peut fonctionner pour être fixé sur un orifice d'accès.

4. Appareil selon la revendication 1, dans lequel l'arbre d'entraînement creux peut fonctionner pour être fixé sur un orifice d'aspiration (28).

5. Appareil selon la revendication 1, dans lequel la seconde extrémité du trocart (19) est couplée à un pignon à rochet (33), le pignon pouvant fonctionner pour aligner les orifices d'échantillonnage du trocart avec les orifices d'échantillonnage de l'arbre d'entraînement creux.

6. Appareil selon la revendication 1, dans lequel le trocart amovible (19) comprend un embout (27) ayant une gorge longitudinale pouvant fonctionner pour libérer des copeaux d'os d'un site d'insertion dans la moelle osseuse, et le perforateur extérieur inclut un bord coupant.

7. Appareil selon la revendication 1, dans lequel le boîtier (12) comprend une portion d'un socle montée sur une surface.

8. Appareil selon la revendication 1, dans lequel l'arbre d'entraînement creux (14) comprend un embout (25) présentant des filetages internes (26) pouvant fonctionner pour s'engager sur un spécimen de biopsie d'os ou de moelle osseuse alors qu'il est retiré de la moelle osseuse.

9. Appareil selon la revendication 1, dans lequel le trocart amovible (19) comprend un embout (27) présentant deux pièces longitudinales séparées qui peuvent se déplacer ensemble ou séparément.

10. Appareil selon la revendication 1, comprenant en outre un ensemble d'engrenages réducteur pouvant fonctionner pour faire tourner l'arbre d'entraînement creux (14).

11. Appareil selon la revendication 1 ou la revendication 16, dans lequel l'arbre d'entraînement creux (14) comprend au moins un palier de butée (21).

12. Appareil selon la revendication 1, dans lequel l'arbre d'entraînement creux (14) comprend au moins un orifice latéral (23a) pouvant fonctionner pour avoir accès à une portion d'os ou de moelle osseuse.

13. Appareil selon la revendication 1, dans lequel le trocart amovible (19) comprend au moins un orifice latéral (13) pouvant fonctionner pour s'aligner avec un orifice latéral (23a) de l'arbre d'entraînement creux (14) pour avoir accès à une portion d'os ou de moelle osseuse.

14. Appareil selon la revendication 1 ou la revendication 16, dans lequel l'arbre d'entraînement creux (14) comprend un connecteur de luer lock (4).

15. Appareil selon la revendication 1, dans lequel le trocart interne (19) comprend deux corps longitudinaux, les corps longitudinaux présentant des embouts munis de mâchoires pouvant fonctionner pour saisir une portion d'os ou de moelle osseuse et dans lequel les corps longitudinaux peuvent fonctionner pour se séparer ou se réunir en fonction de leur position par rapport à l'arbre d'entraînement creux (14).

16. Ensemble d'arbre d'entraînement creux pouvant fonctionner pour retirer de l'os ou de la moelle osseuse d'un os (2) à des fins de diagnostic ou thérapeutiques comprenant :
un arbre d'entraînement creux extérieur (14), et un trocart intérieur amovible (19) pouvant fonctionner pour pénétrer dans la moelle osseuse ;
l'arbre d'entraînement creux extérieur étant couplé de manière amovible à un ensemble d'engrenages (37) ; et
l'arbre d'entraînement creux extérieur comprenant en outre un canal intérieur (15) pouvant fonctionner pour transporter des portions d'os et de moelle osseuse ; **caractérisé par le fait que** le trocart amovible comprend un canal intérieur pouvant fonctionner pour transporter des portions d'os et de moelle osseuse.

17. Ensemble d'arbre d'entraînement creux selon la revendication 16, dans lequel l'arbre d'entraînement creux (14) comprend un embout (25) muni de dents crantées, l'arbre d'entraînement creux comprenant en outre des filetages internes (26) pouvant fonctionner pour s'engager sur une portion d'os ou de moelle osseuse.

18. Ensemble d'arbre d'entraînement creux selon la revendication 16, dans lequel l'arbre d'entraînement creux (14) comprend un corps muni d'au moins un orifice latéral (23 a) pouvant fonctionner pour obtenir des portions de moelle osseuse.

19. Ensemble d'arbre d'entraînement creux selon la revendication 16, dans lequel le trocart intérieur (19) comprend un embout (27) muni d'un bord coupant et comprend en outre un corps muni d'au moins un orifice latéral (13) pouvant fonctionner pour obtenir du tissu de moelle osseuse à partir de la moelle osseuse.

20. Ensemble d'arbre d'entraînement creux selon la revendication 16, dans lequel le trocart intérieur (19) comprend :
deux corps longitudinaux, les corps longitudinaux comprenant en outre des embouts munis de mâchoires incurvées ; et
les corps longitudinaux pouvant fonctionner pour se séparer ou se réunir en fonction de leur position par rapport au perforateur extérieur, et pouvant en outre fonctionner pour saisir une portion d'os ou de moelle osseuse entre les mâchoires incurvées.
